Europäisches Patentamt

European Patent Office

Office européen des brevets

(11) Publication number: **0 286 264**
**A2**

(12) **EUROPEAN PATENT APPLICATION**

(21) Application number: 88302501.7

(22) Date of filing: **22.03.88**

(51) Int. Cl.⁴: **G01N 33/569 , G01N 33/576 , C12N 15/00**

(30) Priority: **23.03.87 US 29298**

(43) Date of publication of application:
**12.10.88 Bulletin 88/41**

(84) Designated Contracting States:
**AT BE CH DE ES FR GB IT LI LU NL SE**

(71) Applicant: **ORTHO PHARMACEUTICAL CORPORATION**
**U.S. Route 202 P.O. Box 300**
**Raritan New Jersey 08869-0602(US)**

(72) Inventor: **Geltosky, John E.**
**4212 Biddeford Circle**
**Doylestown, PA 18901(US)**
Inventor: **Smith, Richard S.**
**12790 Via Donada**
**Del Mar, CA 92104(US)**

(74) Representative: **Jones, Alan John et al**
**CARPMAELS & RANSFORD 43 Bloomsbury Square**
**London, WC1A 2RA(GB)**

(54) **Assay for detecting hepatitis antigens and aids antibodies.**

(57) An assay is provided for detecting whether or not either of the HBsAg or HIV antibody is present in a suspected fluid. A first reagent is provided comprising a solid surface having bound thereto a peptide having an epitope capable of binding to a paratope of a HIV antibody and a solid surface having bound thereto an antibody specific HBsAg.

EP 0 286 264 A2

# ASSAY FOR DETECTING HEPATITIS ANTIGENS AND AIDS ANTIBODIES

## BACKGROUND OF THE INVENTION

This invention relates to a test for the simultaneous detection of markers for the hepatitis B virus and the human immunodeficiency virus (HIV) in a body fluid and in particular in a blood sample. Specifically, the invention relates to a test wherein a single sample of blood may, in a single test, be assayed for the presence of markers for either virus.

The human immunodeficiency virus (HIV) is the etiological agent of the acquired immune deficiency syndrome (AIDS). This retrovirus destroys selected classes of T-lymphocytes resulting in progressive, severe immunodeficiency. Populations at high risk for AIDS are also at risk for hepatitis B (see Rustgi V.K., et al. "Hepatitis B virus Infection in the Acquired Immunodeficiency Syndrome" Annals of Internal Medicine 1984; 101:795-97). Enzyme linked immunosorbent assays (ELISAs) to the surface antigen of hepatitis B (HBsAg) and antibody to HIV are used routinely to monitor donated blood and blood products to prevent transmission of these serious infections by the blood products (See Saxinger W.C., Gallo R.C., "Applications of the Indirect Enzyme-Linked Immunosorbent Assay Microtiter to the Detection and Surveillance of Human T-cell Leukemia-Lymphoma Virus (HTLV)" Laboratory Investigation 1983; 49:371-77 and Wei R., et al., "Solid Phase Enzyme Immunoassay for Hepatitis B Surface Antigen", Clin. Chem. 1977; 23:813-15).

Generally, the assays for antibody to HIV (as well as to a group of closely related retroviruses normally known as Lymphoadenopath Virus, LAV; or AIDs-Related Viruses, ARV) have relied for detection of the antibodies on the use of viral proteins obtained from cultured infected T-lymphocytes. The virus obtained from the cultured cells is disrupted (e.g. with detergent) and a fluid, viral lysate, is obtained. This lysate, which contains a variety of fragments of viral protein, is then typically used as the solid phase component of an immunoassay.

The currently employed commercial immunoassays are of the conventional sandwich ELISA format, in which the solid Phase component, having the viral lysate deposited thereon, is contacted with blood or serum suspected of containing the HIV antibodies. If the antibodies are present, they will bind to the viral lysate and, after unbound material is washed away, the bound antibodies are then contracted with enzyme-labeled anti-human immunoglobulin. The labeled antibodies will bind to any human antibodies attached to the solid phase thus indicating the presence of the bound target HIV antibodies. This type of commercial HIV antibody test is currently used to screen essentially all blood products and has significantly diminished the transmission of HIV virus via employment of such products.

Similarily, blood products are routinely tested for HBsAg by using ELISA assays wherein monoclonal antibodies specific to HBsAg ($\alpha$-HBsAg) are affixed to a solid surface and then contacted with the fluid suspected of containing the target antigens. Unbound material is washed away and the presence of bound antigen is detected by contacting the solid surface with a labeled $\alpha$-HBsAg reagent. After washing and developing the label will indicate the presence of the target antigen.

While, as in the case of the assays for detecting HIV antibodies, such HBsAg assays have been found to be effective in screening blood products, the necessity for performing two separate tests has been both time consuming and inconvenient. This notwithstanding, it is believed that heretofore no effective single test is available which can combine the detection of HBsAg and HIV antibodies in a single assay.

## SUMMARY OF THE INVENTION

In accordance with the teachings of this invention an assay is provided wherein the presence of either HBsAg or HIV antibodies may be detected by combining a suspected fluid sample with a single reagent, incubating the same, washing, and then detecting the target with a labeled reagent.

The combined assay of this invention take advantage of the newly discovered alternative to viral lysate reagent in assaying for HIV antibodies. These new reagents are described in EP-A-87902905.

As described in this patent application , a series of peptides each taken alone or taken in combination may be employed in an ELISA assay to replace the undesirable viral lysate now in commericial use. It is said in such applications that the substitution of the chemically synthesized peptides for the viral lysate is advantageous in that the lysate is produced from live virus infected cells and hence the manufacture and use of such presents the danger of surviving live virus infecting all who come in contact therewith. Further, the viral lysate, being a natural product, is variable which variations may result in unpredictable assays made therewith. Such variation does not exist in the case of chemically synthesized peptides. Finally, it is said that the use of viral lysate in ELISA assays resulted in a substantial number of false positive results whereas, the use of the synthetic peptides has virtually eliminate the same.

It has now been discovered that still another great advantage occurs to the use of such synthetic peptides. Specifically it has been discovered that such peptides may be employed in combination with the HBsAg detecting reagents in a single ELISA assay to simultaneously detect both the HBsAg and the HIV-antibody.

Specifically, an assay for detecting whether or not either of the HBsAg or HIV antibody is present in a suspected fluid is provided which comprises a first reagent. The first reagent comprises: (a) a solid surface having bound thereto a peptide having an epitope capable of binding to a paratope of an HIV antibody, and (b) a solid surface having bound thereto an antibody specific for HBsAg. The assay further includes a second reagent which comprises: (c) a labeled peptide having an epitope capable of binding to a paratope of an HIV antibody, and (d) labeled antibody specific for HBsAg.

In use, the suspected fluid may be brought into contact with the first reagent for a sufficient time to allow an immunologic reaction to occur. Thereafter, the unbound material may be washed from the solid surfaces and then, the solid surface may be contacted with the second reagent. After washing, the presence or absence of labeled second reagent on the solid surface will indicate the presence or absence of the target material (HBsAg or HIV antibody) in the suspected fluid.

In a preferred embodiment the peptide is selected as one or more of the peptides described in the above-mentioned EP-A-87902905.6. In an embodiment of choice, the peptides are chosen as a mixture of two peptides designated and described herein as E32 and E34.

In a preferred embodiment of this invention, the solid surfaces of both (a) and (b) as referred to above are simply the same surface of a well of a microtiter plate.

## DETAILED DESCRIPTION OF THE INVENTION

The invention comprises affixing peptides specific for HIV antibody and antibodies specific to HBsAg to solid surfaces as a first reagent.

The peptides chosen are chemically synthesized oligopeptides having amino acid residues sequences of the critical regions of envelope glycoprotein gp 41 of the HIV. The sequences and synthesis is well described in the EP-A-87902905.6 described above. Specifically, suitable peptides may be selected from the group having the following amino acid residue sequences:

CSGKLIC (I)  
IWGCSGKLICTTAVP (II)  
IWGCSGKLICTTAVPWNAS (III)  
AVERYLKDQQLLGIWGCSGKLI (IV)  
AVERYLKDQQLLGIWGCSGKLICTTAVPWNAS (V)  
LKDQQLLGIWGCSGKLI (VI)  
LLGIWGCSGKLIC (VII)  
QQLLGIWGCSGKLICTTAVPWNAS (VIII)  
IWGCSGKLICTTAVPWN (IX)  
CSGKLICTTAVPWNAS (X)  
SGKLICTTAVPWNAS (XI)  
AVERYLKDQQLLGIWGCSGKLIC (XII)  
GCSGKLICTTAVPWN (XIII)  
LKDQQLLGIWGCSGK (XIV)  
RILAVERYLKDQQLLGIWGCS (XV)

The structure of the subject peptides are given in the conventional singly-letter codes for amino acids. For the convenience of the reader, the single letter codes for the amino acids contained in the subject peptides are:

I = L-isolucine: W = L-tryptophan; G = glycine; C = L-cysteine; S = L-serine; K = L-lysine; L - L-leucine; T - L-threonine; A - L-alanine; V - L-valine; P - L-proline; D = L-aspartic acid; N = L-asparagine; E = L-glutanic acid; R = L-arginine; Y = L-tyrosine; Q = L-glutanine, F = L-phenylalanine; M = L-methionine.

As described in EP-A-87902905.6, recognition of antibodies to HIV is significantly enhanced if the above peptides are used in combination, conforming to a specific selection perscription. Specifically, enhanced recognition is achieved by selecting two or more of the above described peptides wherein a first of said peptides contains the gp 41 protein sequence:

CSGKLIC     (I)

Preferably this first peptide contains at least these seven amino acid residues and still more preferably at least a twelve amino acid residue sequence from the gp 41 protein and including the sequence of formula (I).

The combination of peptides should also include at least a second peptide wherein said peptide includes at least the amino acid residue sequence of

LLG(X)W     (XVI)

wherein X is selected from the group consisting of I, L, M or F. It should be noted that X corresponds to the 602 position of the gp 41 protein and when X is I, the formula (XVI) sequence corresponds to the 599 to 603 position of the gp 41 protein. On the other hand, it is believed that based on a study of the sequence comparison of HIV isolates in the critical region of the gp 41 proteins, substitution for I in this position, 602, with L, M or F are antigenetically equivalent. This second peptides, in addition to including the sequence of formula (XVI), should also include a total of at least fifteen amino acid residues in a sequence found in the gp 41 protein. Stated in other words, the second peptide comprises the sequence

ZLLGXWZ':

wherein X is selected from the group consisting of I, L, M or F;

wherein Z is selected from the amino acid residue sequence of the HTLV-III virus gp 41 protein immediately adjacent to the amino side of the L-leucine residue in the 599th position of the gp 41 protein:

wherein Z' is selected from the amino acid residue sequence of the HTLV-III virus gp 41 protein immediately adjacent to the carboxy side of the L-tryptophan residue in the 603rd position of the gp 41 protein; and

wherein one of Z or Z' may be zero residues long and wherein Z and Z' together comprise at least ten residues.

The combination of peptides (IV) or (XII) with peptides (III), (XIII), or (X) are especially preferred. The combination of (III) and (IV) or (XIII) and (IV) being the combinations of choice.

.The peptides of choice are a mixture of
IWGCSGKLICTTAVPWNAS, hereinafter "E32" and
AVERYLKDQQLLGIWGCSGKLI, hereinafter "E34".

These peptides may be synthesized by the following methods:

## Synthesis of E32 and E34

Synthesis of the peptides E32 and E34 was accomplished using classical Merrifield technique (2). The peptide sequences were synthesized on a Vega 250C automated peptide synthesizer using a double couple program.

For peptide E32, Boc-serine resin with substitution of 0.92 Mmole/gram was used. For peptide E34, Boc-isoleucine resin with substitution of 0.8 Mmole/gram was used. Synthesis of the Boc-serine and Boc-isoleucine resins was accomplished by the Gisin method as described by Stewart & Young (1).

For peptide E32 the peptide sequence 'IWGCSGKLICTTAVPWNAS' was synthesized using the Boc-serine resin sequentially double coupled with the following Bol-L-amino acids in twelve meq excess:

| Amino Acid | Solvent |
|---|---|
| Boc-Ala | $CH_2Cl_2$ |
| Boc-Asn/Hobt | DMF |
| Boc-Trp | 10% $DMF/Ch_2Cl_2$ |
| Boc-Pro | $CH_2Cl_2$ |
| Boc-Val | $CH_2Cl_2$ |
| Boc-Ala | $CH_2Cl_2$ |
| Boc-(O-Bzl)-Thr | $CH_2Cl_2$ |
| Boc-(O-Bzl)-Thr | $CH_2Cl_2$ |
| Boc-(MeOBzl)-Cys | $CH_2Cl_2$ |
| Boc-Ile | $CH_2Cl_2$ |
| Boc-Leu | 10% $DMF/CH_2Cl_2$ |
| Boc-(Cl-Z)-Lys | $CH_2Cl_2$ |
| Boc-Gly | $CH_2Cl_2$ |
| Boc-(O-Bzl)-Ser | $CH_2Cl_2$ |
| Boc-(MeOBzl)-Cys | $CH_2Cl_2$ |
| Boc-Gly | $CH_2Cl_2$ |
| Boc-Trp | 10% $DMF/CH_2Cl_2$ |
| Boc-Ile | $CH_2Cl_2$ |

For peptide E34 the peptide sequence
'AVERYLKDQQLLGIWGCSGKLI' was synthesized using the following Boc-amino acids in 12 meq excess:

5

| Amino Acid | Solvent |
|---|---|
| Boc-Leu | 10% DMF/$CH_2Cl_2$ |
| Boc-(Cl-Z)-Lys | $CH_2Cl_2$ |
| Boc-Gly | $CH_2Cl_2$ |
| Boc-(O-Bzl)-Ser | $CH_2Cl_2$ |
| Boc-(MeOBzl)-Cys | $CH_2Cl_2$ |
| Boc-Gly | $CH_2Cl_2$ |
| Boc-Trp | 10% DMF/$CH_2Cl_2$ |
| Boc-Ile | $CH_2Cl_2$ |
| Boc-Gly | $CH_2Cl_2$ |
| Boc-Leu | 10% DMF/$CH_2Cl_2$ |
| Boc-Leu | 10% DMF/$CH_2Cl_2$ |
| Boc-Gln/Hobt | DMF |
| Boc-Gln/Hobt | DMF |
| Boc-(Bzl)-Asp | $CH_2Cl_2$ |
| Boc-(Cl-Z)-Lys | $CH_2Cl_2$ |
| Boc-Leu | 10% DMF/$CH_2Cl_2$ |
| Boc-(Br-Z)-Tyr | $CH_2Cl_2$ |
| Boc-(Tosyl)-Arg | 10% DMF/$CH_2Cl_2$ |
| Boc-(Bzl)-Glu | $CH_2Cl_2$ |
| Boc-Val | $CH_2Cl_2$ |
| Boc-Ala | $CH_2Cl_2$ |

(1) Stewart & Young, Solid Phase Peptide Synthesis, 2nd edition (1984), Pierce Chemical Co.

(2) Merrifield, R.B. (1963), J. Amer. Chem. Soc. 85, pp. 2149-2154

(3) All Boc-amino acids obtained from Bachem Inc., Torrance, Calif.

(4) Analytical HPLC Conditions:

Buffer A: 0.1% TFA/Distilled Deionized Water

Buffer B: 0.1% TFA/HPLC grade Acetonitrile

Gradient Conditions: 10% 'B' to 50% 'B' over 20 minutes

Wavelength: 214nm

Flow: 1.0 ml/min

Column: Vydac 214TP54 C-4 Protein column 250 $\times$ 4.5 mm

(5) Amino Acid analysis performed on a LKB 4150 ALpha Amino Acid Analyzer.

(6) Peptide sequence determination performed on an Applied Biosystems 470A Protein Sequencer.

(7) Peptide content determined from recovery on amino acid analysis of known amount of peptide.

(8) Boc is a chemical abbreviation for the tert-Butyloxcarbonyl alpha-amino protecting group. The functional group is removed by hydrolysis in 50% Trifluoroacetic acid (TFA)/50% Dichloromethane ($CH_2Cl_2$) after the amino acid has been coupled to the growing peptide chain. This action exposes the amino terminus of the chain to allow the next amino acid to be effectively coupled.

In addition to the Boc protecting group on every amino acid, the side chains of some amino acids are further protected from attack by the chemistry of peptide synthesis. These protecting groups, listed in parenthesis on the accounting list, are all stable to the conditions of peptide synthesis, yet are easily removed from the amino acid during cleavage in hydrofluoric acid. Anisole (methylphenyl ether) acts as a nucleophilic scavenger during the HF cleavage step to prevent alkylation of the peptide by the liberated protecting group carbonium ions. The protecting groups for the amino acids listed are defined below:

O-Benzyl: Benzyl-ester; attached to the hydroxyl side chain of both serine and threonine to prevent the acylation or branching of the peptide chain.

MeObzl: 4-Methoxybenzyl; attached to the sulfhydryl group of cysteine to prevent its oxidation during peptide synthesis.

Cl-Z: 2-chlorobenzyloxycarbonyl; attached to the alpha-amino group of lysine to prevent the formation of side chain growth from this site on the peptide.

Hobt: 1-hydroxybenzotriazole; used in equimolar amounts to glutamine and asparagine during coupling to prevent dehydration to the nitrile forms.

Tosyl: p-toluene sulfonyl; used to acylate the guanidine group in the side chain of arginine.

Bzl: beta-benzyl ester; blocks the carboxyl groups in the side chain of aspartic acid and glutamic acid.

BrZ: 2-bromobenzyloxycarbonyl; blocks the hydroxyl group in the side chain of tyrosine.

Peptides were cleaved from the resin, filtered, extracted with acetic acid and run through a Fractogel desalting column as in Example I. For peptide E34, Fractogel fractions were analyzed by analytical HPLC and fractions containing at least 30% of the total absorption at 214 nm as the major peak migrating at approximately 14 minutes retention time were pooled. The pooled fractions were chromatographed on carboxymethyl cellulose equilibrated with 0.01 M ammonium acetate, pH 4.4. The column was eluted with a step gradient of ammonium acetate and the fraction eluting at 0.2M ammonium acetate was collected, lyophilized, and analyzed by analytical HPLC. The major peak migrating at 14 minutes retention time comprised between 30% and 40% of the total absorption at 214 nm and the material had an acceptable amino acid content. This material was resolubilized and used in ELISA.

For peptide E34, Fractogel fractions were likewise analyzed by analytical HPLC. Fractions containing at least 70% of the total absorption at 214 nm as the major peak migrating at approximately 12.99 minutes retention time were pooled, lyophilized, and analyzed by HPLC and for amino acid content. This material was resolubilized and used in ELISA.

The anti-HBsAg (preferably monoclonal) to be employed with the synthetic protein in the combined assay of this invention may be any such monoclonal antibody shown to be specific for the HBsAg and usable in an ELISA assay. Specifically employed was an anti-HBsAg mouse monoclonal obtained from Ortho Diagnostic Systems. Inc., of Raritan, New Jersey, identical to that used in the antibody to hepatitis B surface antigen (anti-HBs) (murine monoclonal) Ortho antibody to HBsAg ELISA Test System. Other sources of anti-HBsAg monoclonal antibodies are known such as from Accurate Chemical & Scientific Corp (Westbury, NY) (e.g. Product numbers MAS 103. MAS 104, MAS 105, MAS 106, or MAS 107). Alternatively, well known hybridoma technology techniques may be employed to raise, select and purify a suitable monoclonal antibody to HBsAg. Such methods would include, for example, the isolation and purification of hepatitis related antigens, including the viral surface antigens. Similarly, the production of hybridomas pursuant to methods first advanced by Kohler and Milstein [Nature 256, 495-497 (1975)] are well known and need not be repeated here. While monoclonal antibodies are generally preferred due to their great specifity, polyclonal antibodies may also be advantageously employed. Procedures for making polyclonal antibodies are equally well known or, they may be obtained commercially such as from Cambridge Research Laboratories, Massachusetts (eg. Prod 597015 Hepatitis B Surface Antigen goat polyclonal antibody).

In accordance with the teachings of this invention, the first reagent is prepared by affixing both the peptides and the anti-HbsAg monoclonal to solid surfaces. Such solid surfaces may be particles of various materials such as for example polysaccharides, silicaceous materials, or plastics or the like. Preferably, in ELISA assays the solid surfaces are the wells of a microtiter plate well and both the monoclonal and the peptides are affixed to the surface of each well.

The second reagent comprises a mixture of labeled peptides and antibodies. While many such "labels" are known in the art to be coupled to immunologically active materials in ELISA assay, the label of choice for the peptides is biotin-streptavidin-biotin-HRPO (horse radish peroxidase) complex. The label of choice for the antibodies is HRPO.

## BASIC OUTLINE OF PREFERRED ASSAY

The basic outline of a preferred assay of this invention is as follows:

1) The first reagent, comprising both E32 and E34 peptides and α-HBsAg monoclonal coated to the surface of a well of a microtiter plate, are incubated with the suspected fluid e.g., patient plasma, for sufficient time to immunoreact e.g. for one hour at 37°C and wash;

2) The second reagent, comprising E32 and E34 coupled with biotin and in mixture with anti-HBsAg coupled to HRPO, is added and incubated for sufficient time to immunoreact e.g. one hour at 37°C, then wash;

3) The label is developed by adding a prepared streptavidin biotin-HRPO complex and allowing incubation to bound peptide-biotin complex e.g. for thirty minutes at 37°C;

4) Finally the label is developed with OPD substrate in thirty minutes at room temperature and the optical density is read at 492 nm.

## MATERIAL AND METHODS

### Study Subjects and Control Groups

One group of 30 serum specimens was obtained from patients with AIDS or AIDs-related complex (ARC) from San Diego. 48 specimens were obtained in February and March, 1980, as part of an epidemiologic study of hepatitis B in homosexual men. Serum was stored at -20 C. These sera were initially screened for HBV surface antigen and for antibody to it by the AusRIA kits (Abbott Laboratories, North Chicago, Ill). A total of 32 serum samples from low risk patients in a Gastroenterology Clinic were assayed as unknowns in the anti-HIV env/HBsAg assay. In addition, 26 sera from patients with diseases due to rheumatoid arthritis, systemic lupus erythematosus and trauma unrelated to HIV infection were assayed.

### E32'E34 env Peptides and ELISA Using Them as Antigens

E32 and E34 are synthetic oligopeptides from regions of envelope glycoprotein gp 41 of HIV. Their sequence, their synthesis and the ELISA using them as antigen has previously been described.

### Biotinylation of env Peptides

The HIV env peptides E32 and E34 used in this study were synthesized with the solid phase method by Merrifield [6]. The peptide sequences were characterized by amino acid analysis (LKB 4150 amino acid analyzer) and amino acid sequence (Applied Biosystems 470 H protein sequences). Analytical HPLC of the pooled proteins showed the peptide to be greater than 70% pure. The peptide was lyophilized to remove acetic acid.

A quantity of 1.0 mg of E32 and E34 peptides were solubilized separately in 250 ul 0.1 M sodium carbonate-bicarbonate buffer,. pH 9.6 on ice.

In separate test tubes, 0.5 mg biotin-x-NHS (biotinyl-e-aminocaproic acid n-hydroxy-succinimide ester, Calbiochem, San Diego, CA) for E32, and 0.1 mg biotin-x-NHS for E34, were dissolved in 1.0 ml of 0.1 M sodium phosphate (0.1 M $NaH_2PO_4$. 0.1 M $Na_2HPO_4$, pH 6.5) on ice. To each test tube 1.0 mg of EDC (1-ethyl-3-(3-dimethylaminopropyl) carbodiimide-HCL, Pierce Chemical Co., Rockford, ILL) was added and the solution incubated for 15 minutes on ice. A volume of 750 ul of 0.1 M sodium phosphate was added to the peptide solutions which were then added separately to the appropriate EDC-biotin-x-NHS solution to a total volume of 2 ml. The test tubes were placed on an end-over-end rotator and incubated for 30 minutes at 4C, and then 4 hours at room temperature.

The biotinylated peptide solutions were chromatographed on a Sephadex G-10 (Pharmacia Fine Chemicals, Piscataway NJ) 1.0 $^\times$ 20 cm column (Biorad Laboratories, Richmond, CA). with a bed volume of 10.0 ml. The peptides were eluted with PBS PH 7.2 at a flow rate of 1.0 ml/min.

The absorbance was measured at 235 nm with PBS as the reference blank. The major peak of absorbance at 235 nm was collected, pooled, and frozen at -20C with 10% glycerol added to stabilize the peptides.

The efficacy of the biotinylation was monitored by running various dilutions of the biotinylated peptides in the anti-HIV env peptide/HBsAg assay to determine which dilution resulted in the most distinct ratio of signal between positive and negative samples.

Anti-HIV env Peptide/HBsAg ELISA

The E32 and E34 peptides were solubilized by dissolving the peptides in 0.1M sodium carbonate-bicarbonate buffer, pH 9.6 to a concentration of 200 ug/ml. The ELISA assay was performed on "removawell" 12 well microtiter strips (Nunc, Irvine Scientific, Irvine, CA. or Dynatech Immulon II, Chantilly, Va.). Each well was coated with a solution of 200 ul of 2.5 ug/ml E32 and 0.5 ug/ml peptide 34. Each well was also coated with 10 ug/ml of anti-HBsAg mouse monoclonal. (Ortho Diagnostics Systems, Inc., Raritan, NJ) in the same buffer. The microtiter plate was covered with parafilm and incubated for a minimum of 12 hours at 4C.

The env peptide-anti HBsAg solution was then discarded and excess binding sites quenched with 300 ul/well of 10% normal horse serum in PBS (0.15 M NaCl, 0.05 M $NaH_2PO_4$. 0.05M $Na_2HPO_4$, pH 7.3) to which 2.5% fetal calf serum, 0.2% bovine serum albumin (BSA) and 0.05% Tween-20 had been added. This solution was incubated for 15 minutes in a 37°C humidified chamber. The wells were emptied by inversion and 200 ul/well of neat plasma or serum was added to each test well. The microtiter plate was covered with parafilm, incubated for 1 hour at 37C, then washed 5 times with 400 ul/well of 0.05% Tween-20 in PBS.

A volume of 200 ul was then added to each well of a solution in PBS containing 1:50 mouse monoclonal to HBsAg conjugated to HRPO and a 1:350 dilution of each biotinylated peptide. After one hour incubation at 37 C, the wells were washed 5 times with 400 ul of 0.05% Tween-20 in PBS.

A solution of 200 ul/well streptavidin-biotin-horseradish peroxidase complex, previously incubated for 30 minutes at 37°C (Zymed Laboratories, San Francisco, CA) was added to each well and incubated for 30 minutes at 37C. The wells were washed 5 times with 0.05% Tween-20 in PBS and incubated with 200 ul/well of o-phenylenediamine solution (one-10 mg OPD tabled in 15 ml distilled water and 6.25 ul 30% $H_2O_2$; Sigma Chemical Co., St. Louis, MO) for 30 minutes at room temperature. The reaction was stopped by the addition of 50 ul/well of 4N $H_2SO_4$. Absorbance at 490 nm was determined in a microelisa reader zeroed on an empty microtiter well.

Evaluation of Anti-HIV env Peptide/HBsAg ELISA

The intra and inter assay reproducibility of the anti-HIV env/HBsAg assay was determined by measuring three dilutions of two separate samples (one HIV positive and one HBsAg positive) within the same assay or within multiple assays performed several times during a week.

The recovery of various proportions of a HIV positive sample added to a HIV negative sample and proportions of a HBsAg positive control added to a HBV negative serum were determined. These supplemental and unsupplemented samples were assayed in the anti-HIV env/HBsAg as unknowns.

Reference HIV Antibody and HBsAg procedures

Antibody to HIV in serum and plasma was measured with two commercially-available ELISA kits: Virgo HTLV-III ELISA, (Electro-Nucleonics, Inc., Columbia, MD), henceforth called HIV ELISA I: and Abbott HTLV III EIA (Abbott Laboratories, North Chicago, Ill), henceforth called HIV ELISA II. Hepatitis surface antigen was measured by the Auszyme HBsAg EIA Kit (Abbott Laboratories, North Chicago, ILL), called HBsAg I, and the Ortho HBsAg ELISA Test System, (Ortho Diagnostic Systems. Inc., Raritan. NJ) termed HBsAg II. These assay procedures were performed according to the manufacturer's instructions.

Standards

For HBsAg sensitivity comparison we used the Abbott Laboratories HBsAg Standard (Lot #84464HR) subtypes ad and ay The standards were stored at 4C and assayed in duplicate.

## Immunoblot Assay for HIV

Selected samples were analyzed by immunoblot analysis as described by Symington [See Symington, J. et al., "Immunoautoradiographic Detection of Proteins after Electrophoretic transfer from Gels to Diazo-Paper. Analysis of Adenovirus Encoded Proteins", Proc. Natl. Acad. Sci. 1981;78:177-181] using the BioRad immunoblot assay, (Bio-Rad Clinical Division. Richmond, CA). A specimen was considered to be reactive by the immunoblotting technique by the presence of antibody directed to both the 24 KD gag and 41 KD env glycoprotein bands. This Bio-Rad assay procedure was performed according to the manufacturer's instructions.

## Statistical Analyses

Mean values are expressed with their standard deviations. Outlying normal values were removed from the normal range studies using the statistical theory described by Barnett [See Barnet R.M., "Clinical Laboratory Statistics", 1979 2nd Ed. Boston. Little, Brown & Company 124].

## Results

## Normal Range Determinations and Control Groups

Serum or plasma was obtained from 72 healthy volunteers and assayed in duplicate in the HIV env peptide/HBsAg assay. Two samples were rejected as statistically significant outlyers because of optical density values (0.62 and 0.054) above the normal range determined according to the theory of Barnett. These sera were verified to be negative for HBsAg, and HIV by commercial ELISA and Western Blot. The normal range for the anti HIV env/HBsAg ELISA was then calculated from the assay results of testing 70 samples. The mean optical density value or absorbance was 0.008. The mean optical density value plus six standard deviations was 0.096. The anti-HIV env peptide/HBsAg ELISA results on unknown sera above an optical density of 0.100 were considered to be positive. The mean optical density or absorbance, when the values from the two outlyers were added to the distribution, was determined to be 0.026. This mean plus three standard deviations was 0.097 and plus six standard deviations was 0.168. The 32 samples from the Gastroenterology Clinic were negative in the anti-HIV env/HBsAg assay as were the 26 other samples from miscellaneous diseases.

For the anti-HIV env/HBsAg assay, the intra-assay coefficient of variation was determined by assaying three different plasma samples 8 times in a single assay and ranged from 3.0 to 7.0. The inter-assay coefficient of variation was established by assaying three plasma pools on three consecutive days and ranged from 3.0 to 11.0 (table I). To assess the accuracy of this assay, we performed plasma supplement and recovery experiments in which dilutions of a HIV positive and a HBsAg positive control were added separately to a normal plasma, which was then assayed in the anti-HIV env/HBsAg assay. The results for the HIV antibody recovery experiment demonstrate that the anti-HIV env/HBsAg ELISA could detect the various dilutions (1:2-1:200) of a HIV positive specimen added to a HIV negative specimen. Known amounts of a HBsAg positive control was added to a normal serum and assayed in the anti-HIV env/HBsAg ELISA. The results indicated that the anti-HIV env/HBsAg assay can detect various concentrations from 6 nanograms to 0.75 nanograms of this HBsAg control added to normal serum.

## Comparison of the Anti-HIV env/HBsAg Assay With HIV and HBsAg ELISA Kits

Specimens obtained from 48 homosexual men obtained in early 1980 were assayed in duplicate in the anti-HIV env/HBsAg ELISA and compared to HIV and HBsAg ELISA procedures as described in the Materials and Methods. The results demonstrated that the anti HIV env/HBsAg ELISA accurately detected antibodies to HIV and HBV surface antigen in specimens when one or both of the analytes were present (table II). The strongest signal in terms of absorbance was observed with samples that were HBsAg positive alone. A total of 6 specimens were E32/E34 ELISA test positive and negative in the anti HIV env/HBsAg assay. As an additional test of specificity of the anti HIV env/HBsAg ELISA, 30 specimens from patients with AIDS or ARC that had been screened for the presence of HBsAg were assayed in the anti-HIV env peptides HBsAg and HIV kits. The results (table III) demonstrate complete concordance between test results and the HIV and HBsAg test kits. The anti-HIV env peptide/HBsAg assay detected all samples as positive that contained HBsAg alone, antibodies to HIV alone, or both.

Sensitivity for HBsAg

Dilutions of HBsAg standards containing a known quantity of HBsAg were assayed in the anti-HIV env peptide/HBsAg ELISA and compared to HBsAg ELISA methods #I and II. The results of this study (table IV) show that the anti-HIV env peptide/HBsAg ELISA was as sensitive as the reference procedures for the detection of known amounts of HBsAg from standards of subtypes ad and ay. The HBsAg ELISA method #II was able to detect one more dilution of the ad and ay standard or less than 1 nanogram of sensitivity compared to HBsAg ELISA Method #1.

Discussion

It can be seen that what is provided by this invention is an ELISA that can detect simultaneously samples with either antibody to HIV or HBV surface antigen. The antibody to HIV was detected with synthetic oligopeptides used as the antigen instead of HIV viral lysates. A sandwich peptide assay was established when the bivalent human HIV antibody on the solid phase was incubated with biotinylated peptide env after the primary reaction with sera. The bound biotinylated peptide was then detected by a streptavidin-biotin-HRPO complex. This ELISA has comparable sensitivity to commercial procedures for the detection of antibody to HIV. A complementary, non-competing pair of monoclonal antibodies form the specific detection system for HBsAg. One of these antibodies is conjugated with HRPO to form the enzyme detection system. Due to the complexity of both immunochemical systems in one reaction vessel, an unexpected advantage of the anti-HIV env/HBsAg was the presence of low ELISA backgrounds. These low backgrounds were due in part to the specificity of the well defined HIV env peptides used in this assay instead of HIV viral lysate and HIV specific recombinant proteins. HIV env assay sensitivity can be enhanced by increasing the molar concentration of the env epitope on the solid phase a problem with ELISAs formatted with HIV viral lysates. The anti-HIV env/HBsAg assay was used to detect the presence of HIV antibody and HBsAg in 60 samples that have serological evidence of infection due to HIV or HBV. The anti-HIV env/HBsAg assay detected one or both analytes in all studied samples.

The one disturbing result noted in these studies was the 6 E32/E34 ELISA false positives that were assay negative in the anti-HIV env/HBsAg assay. These samples were also negative in two commercial HIV ELISA procedures. Where volume of sample would allow, four of these samples were determined to be negative by immunoblots. It is speculated that these E32/E34 ELISA false positives may be assay or format specific. These samples were negative in the anti-HIV env/HBsAg assay which would support this hypothesis.

## Table I

### Intra and Interassay Variability of the Anti-HIV env/HBsAg Assay

|  | Plasma 1 | | Plasma 2 | | Plasma 3 | |
|---|---|---|---|---|---|---|
|  | HBsAg(+) | HIV(+) | HBsAg(+) | HIV(+) | HBsAg(+) | HIV(+) |
| **Intra-assay** | | | | | | |
| No. of determinations | 8 | 8 | 8 | 8 | 8 | 8 |
| mean $OD_{490}$ | 1.33 | 1.519 | .948 | 1.350 | .656 | 1.106 |
| SD | 0.068 | 0.062 | 0.028 | 0.083 | 0.037 | 0.080 |
| cv | 5.0 | 4.0 | 3.0 | 6.0 | 6.0 | 7.0 |
| **Inter-assay** (n = 3 each) | | | | | | |
| mean $OD_{490}$ | 1.287 | 1.365 | .921 | 1.353 | .728 | .829 |
| SD | 0.042 | 0.153 | 0.024 | 0.102 | 0.090 | 0.064 |
| cv | 3.0 | 11.0 | 3.0 | 7.5 | 12.0 | 8.0 |

Table II
Determination of the presence of HBsAg antigen and antibody to HIV
in sera collected in early 1980 from 48 homosexual men.

| Study Number | Anti-HIV env/ HBsAg Assay | | HIV Tests E32/ E34 ELISA | ELISA #I | ELISA #II | HBV Tests HBsAg | HBsAb | Western Blot |
|---|---|---|---|---|---|---|---|---|
| HBsAg Positive Samples | | | | | | | | |
| 1053 | 2.000 | + | – | – | – | + | + | |
| 1062 | 2.000 | + | – | – | – | + | + | |
| 1151 | 0.250 | + | – | – | – | + | – | |
| 1160 | 1.159 | + | – | – | – | + | – | |
| 1179 | 2.000 | + | – | – | – | + | – | |
| 1185 | 2.000 | + | – | – | – | + | – | |
| 1191 | 2.000 | + | – | – | – | + | – | |
| 1197 | 2.000 | + | – | – | – | + | – | |
| 1216 | 2.000 | + | – | – | – | + | – | |
| 1221 | 2.000 | + | – | – | – | + | – | |
| 1224 | 2.000 | + | – | – | ÷ | + | – | |
| 1228 | 2.000 | + | – | – | – | + | – | |
| 1243 | 2.000 | + | – | – | – | + | – | |
| 1250 | 2.000 | + | – | – | – | + | – | |
| 1269 | 2.000 | + | – | – | – | + | – | |
| 1282 | 2.000 | + | – | – | – | + | – | |
| HIV Positive Samples | | | | | | | | |
| 1090 | 0.486 | + | + | + | + | – | + | |
| 1123 | 0.259 | + | + | + | + | – | + | |
| 1145 | 1.060 | + | + | + | + | – | + | |
| 1147 | 1.356 | + | + | + | + | – | + | |
| 1155 | 0.216 | + | + | + | + | – | + | |
| 1163 | 0.391 | + | + | + | + | – | – | |
| 1182 | 0.504 | + | + | + | + | – | + | |
| 1186 | 0.214 | + | + | + | + | – | + | |
| 1202 | 0.740 | + | + | + | + | – | + | |
| 1234 | 0.677 | + | + | + | + | – | + | |
| 1272 | 0.542 | + | + | + | + | – | + | |
| 1335 | 0.775 | + | + | + | + | | | |
| 1203 | 0.703 | + | + | + | – | – | + | + |
| HBsAg and HIV Positive Sample | | | | | | | | |
| 1120 | 0.525 | + | + | + | + | + | – | |
| E32/E34 ELISA False Positives | | | | | | | | |
| 1085 | 0.000 | – | + | – | qns | – | – | – |
| 1089 | 0.069 | – | + | – | – | – | – | qns |
| 1210 | 0.046 | – | + | – | – | – | + | |
| 1222 | 0.000 | – | + | – | – | – | + | – |
| 1245 | 0.085 | – | + | – | – | – | + | – |
| 1164 | 0.078 | – | + | – | – | – | + | – |
| HBsAg and HIV Negative Samples | | | | | | | | |
| 1063 | 0.000 | – | – | – | – | – | | |
| 1084 | 0.000 | – | – | – | – | – | + | |
| 1091 | 0.059 | – | – | – | – | – | + | |
| 1157 | 0.000 | – | – | – | – | – | – | |
| 1159 | 0.000 | – | – | – | – | – | – | |
| 1161 | 0.000 | – | – | – | – | – | – | |
| 1211 | 0.098 | – | – | – | – | – | + | |
| 1217 | 0.000 | – | – | – | – | – | + | |
| 1233 | 0.093 | – | – | – | – | – | + | |
| 1244 | 0.022 | – | – | – | – | – | + | |
| 1263 | 0.099 | – | – | – | – | – | + | |
| 1271 | 0.098 | – | – | – | – | – | – | |

## Table III

Determination of the presence of HBsAg antigen and antibody to HIV in samples from 30 patients with AIDS or ARC.

| Study Number | Draw Date | Anti-HIV env HBsAg Assay | | HIV E32/E34 ELISA | ELISA Tests #1 | #2 | HBsAg Test #I |
|---|---|---|---|---|---|---|---|
| 001 | 8-19-86 | 0.540 | + | + | + | + | − |
| 002 | 8-20-86 | 0.761 | + | + | + | + | + |
| 003 | 8-1-86 | 2.000 | + | + | + | + | − |
| 004 | 8-19-86 | 1.087 | + | + | + | + | − |
| 005 | 8-12-86 | 2.000 | + | + | + | + | − |
| 006 | 7-8-86 | 2.000 | + | + | + | + | − |
| 007 | 8-8-86 | 2.000 | + | + | + | + | + |
| 008 | 8-14-86 | 2.000 | + | + | + | + | Q[1] |
| 009 | 8-14-86 | 0.627 | + | + | + | + | − |
| 010 | 8-20-86 | 2.000 | + | + | + | + | − |
| 012 | 7-31-86 | 1.197 | + | + | + | + | + |
| 014 | 7-2-86 | 2.000 | + | + | + | + | − |
| 015 | 7-9-86 | 2.000 | + | + | + | + | + |
| 016 | 8-1-86 | 2.000 | + | + | + | + | − |
| 017 | 8-12-86 | 2.000 | + | + | + | + | − |
| 018 | 8-14-86 | 2.000 | + | + | + | + | − |
| 019 | 8-14-86 | 2.000 | + | + | + | + | + |
| 020 | 8-15-86 | 1.992 | + | + | + | + | + |
| 021 | 8-20-86 | 2.000 | + | + | + | + | − |
| 023 | 7-24-86 | 1.659 | + | + | + | + | + |
| 025 | 8-8-86 | 1.109 | + | + | + | + | − |
| 026 | 8-12-86 | 2.000 | + | + | + | + | + |
| 027 | 8-15-86 | 1.671 | + | + | + | + | − |
| 030 | 7-30-86 | 2.000 | + | + | + | + | + |
| 032 | 6-12-86 | 2.000 | + | + | + | + | − |
| 034 | 7-8-86 | 2.000 | + | + | + | + | − |
| 035 | 7-10-86 | 2.000 | + | + | + | + | − |
| 036 | 7-8-86 | 2.000 | + | + | + | + | Q[1] |
| 037 | 7-11-86 | 2.000 | + | + | + | + | + |
| 040 | 8-8-86 | 0.995 | + | + | + | + | − |

[1]Quantity not sufficient for assay

Table IV

Sensitivity of the anti-HIV env/HBsAg assay compared to HBsAg ELISA
Methods #I and II. Individual samples of the Abbott HBsAg standard were
assayed in the three procedures. The cutoff value for the anti-HIV
env/HBsAg assay was 0.100.

| Sample | Assigned Value (ng/ml) | Subtype | Anti-HIV env Peptide/ HBsAg Assay | | HBsAg ELISA #I | | HBsAg ELISA #II | |
|--------|------------------------|---------|-----------|---|--------|---|--------|---|
| A | 2.13 | ad | .161 | + | .084 | + | .105 | + |
| R | 1.40 | ad | .096 | + | .064 | + | .061 | + |
| M | 1.12 | ad | .137 | + | .056 | + | .052 | + |
| U | .87 | ad | .045 | − | .044 | − | .053 | + |
| J | .60 | ad | .063 | − | .041 | − | .033 | − |
| P | .45 | ad | .053 | − | .031 | − | .028 | − |
| D | .31 | ad | .046 | − | .029 | − | .018 | − |
| G | .17 | ad | .022 | − | .026 | − | .020 | − |
| T | .06 | ad | .048 | − | .021 | − | .028 | − |
| B | 1.31 | ay | .112 | + | .064 | + | .100 | + |
| F | 1.09 | ay | .174 | + | .058 | + | .092 | + |
| Q | .88 | ay | .367 | + | .051 | + | .060 | + |
| L | .68 | ay | .033 | − | .041 | − | .051 | + |
| C | .55 | ay | .075 | − | .035 | − | .043 | + |
| H | .47 | ay | .032 | − | .032 | − | .036 | − |
| K +/− | .39 | ay | .041 | − | .029 | − | .028/.043 | |
| S | .30 | ay | .017 | − | .026 | − | .036 | − |

Claims

1. An assay for detecting whether or not either of the HBsAg and HIV antibodies are present in a suspected fluid comprising:

a first reagent comprising
(a) a solid surface having bound thereto one or more peptides having an epitope capable of binding to a paratope of an HIV antibody; and
(b) a solid surface having bound thereto an antibody specific for HBsAg; and
a second reagent comprising
(c) one or more labeled peptides having an epitope capable of binding to a paratope of an HIV antibody; and
(d) labeled antibody specific for HBsAg;

wherein said suspected fluid may be brought into contact with said first reagent, unbound material may be washed from said solid surfaces, the solid surfaces may be contacted with the second reagent and again washed and said labels may be employed to detect the presence or absence of either of HBsAg or HIV antibodies.

2. The assay of claim 1 wherein said peptides include a sequence selected from the group consisting of:

```
CSGKLIC        (I)
IWGCSGKLICTTAVP        (II)
IWGCSGKLICTTAVPWNAS        (III)
AVERYLKDQQLLGIWGCSGKLI        (IV)
AVERYLKDQQLLGIWGCSGKLICTTAVPWNAS        (V)
LKDQQLLGIWGCSGKLI        (VI)
LLGIWGCSGKLIC        (VII)
QQLLGIWGCSGKLICTTAVPWNAS        (VIII)
IWGCSGKLICTTAVPWN        (IX)
CSGKLICTTAVPWNAS        (X)
SGKLICTTAVPWNAS        (XI)
AVERYLKDQQLLGIWGCSGKLIC        (XII)
GCSGKLICTTAVPWN        (XIII)
LKDQQLLGIWGCSGK        (XIV)
RILAVERYLKDQQLLGIWGCS        (XV)
```

the antigenic and immulogic fragments and the diagnostically acceptable salts thereof.

3. The assay of claim 1 wherein either or both of said reagent comprises a mixture of said peptides, said mixture comprising two or more peptides containing amino acid residue sequences homologous to portions of the HIV gp 41 protein sequence wherein in each said mixture:

a first of said peptides comprises the portion of the HIV gp 41 protein sequence CSGKLIC; and

a second of said peptides comprises the sequence ZLLGXWZ';

wherein X is selected from the group consisting of I, L, M or F; Z is selected from the amino acid residue sequence of the HIV gp 41 protein immediately adjacent to the amino side of the L-leucine residue in the 599th position of the gp 41 protein; Z' is selected from the amino acid residue sequence of the HIV gp 41 protein immediately adjacent to the carboxyl side of the L-tryptophan residue in the 603rd positions of the gp 41 protein; wherein one of Z and Z' may be zero residues long; and wherein Z and Z' together comprise at least ten residues.

4. The assay of claim 3 wherein said first peptide is selected from the group consisting of:

IWGCSGKLICTTAVPWNAS;

GCSGKLICTTAVPWN;

CSGKLICTTAVPWNAS; and

said second peptide is selected from the group consisting of:

AVERYLKDQQLLGXWGCSGKLI, and
AVERYLKDQQLLGXWGCSGKLIC.

5. The assay of claim 4 wherein X is I.

6. The assay of any one of claims 1 to 5 wherein said solid surface comprises particles of plastic, sicilaceous particles or polysaccharide particles.

7. The assay of any one of claims 1 to 6 where said surfaces of (a) and (b) both comprise the walls of a single well of a microtiter plate.

8. A method for detecting whether or not either of the HBsAg and HIV antibodies are present in a suspected fluid comprising the steps of:

(a) providing a first reagent comprising a solid surface having bound thereto one or more peptides having an epitope capable of binding to a paratope of an HIV antibody and a solid surface having bound thereto a monoclonal antibody specific for HBsAg;

(b) contacting said first reagent with the suspected fluid for a sufficient time to allow an immunologic reaction to occur; and

(c) detecting the presence of HBsAg or HIV antibodies bound to said solid surfaces.

9. The method of claim 8 further comprising the step of:

providing a second reagent comprising labeled peptide having an epitope capable of binding to a paratope of an HIV antibody and labeled antibody specific for HBsAG; and

wherein the step (c) of detecting the presence of HBsAg or HIV antibodies bound to said solid surfaces is accomplished by washing the solid surfaces free of unbound material after step (b); contacting the washed solid surfaces with the second reagent; washing said second reagent contacted solid surfaces free of unbound material; and developing the label as an indicia of the presence of either of said HBsAg or HIV antibodies.

10. The method of claim 8 or claim 9, wherein said one or more peptides comprise a mixture of peptides; said mixture comprising two or more peptides containing amino acid residue sequences homologous to portions of the HIV gp 41 protein sequence wherein in each said mixture:

a first of said peptides comprises the portion of the HIV gp 41 protein sequence CSGKLIC; and

a second of said peptides comprises the sequece ZLLGXWZ':

wherein X is selected from the group consisting of I, L, M or F; Z is selected from the amino acid residue sequence of the HIV gp 41 protein immediately adjacent to the amino side of the L-leucine residue in the 599th position of the gp 41 protein; Z' is selected from the amino acid residue sequence of the HIV gp 41 protein immediately adjacent to the carboxyl side of the L-tryptophan residue in the 603rd positions of the gp 41 protein; wherein one of Z and Z' may be zero residues long; and wherein Z and Z' together comprise at least ten residues.